# EUROPÄISCHE PATENTANMELDUNG

(11) **EP 2 497 480 A1**
(43) Veröffentlichungstag der Anmeldung: **12.09.2012**
(21) Anmeldenummer: 12158476.7
(22) Anmeldetag: 07.03.2012
(51) Int. Cl.: A61K 35/66, A61K 39/00

(54) **Extrakte aus phototrophen Mikroorganismen als Adjuvans**

(30) Priorität: 07.03.2011 EP 11157187
(71) Anmelder: IGV Institut für Getreideverarbeitung GmbH, 14558 Nuthetal (DE); RIPAC-LABOR GmbH, 14476 Potsdam (DE)
(72) Erfinder: Pulz, Otto, 14558 Nuthetal (DE); Köhler, Eva-Maria, 14478 Potsdam (DE); Storandt, Regina, 14467 Potsdam (DE); Köhler, Bernd, 14478 Potsdam (DE); Metzner, Martin, 14471 Potsdam (DE); Köhler-Repp, Dagmar, 14476 Potsdam (DE)
(74) Vertreter: Hoppe, Georg Johannes

(57) **Zusammenfassung**

Die Erfindung betrifft ein Adjuvans aus einem Extrakt aus einem phototrophen Mikroorganismus, das in Verbindung mit einem Immunstimulator (Antigen) bei einmaliger parenteraler Applikation die aktive spezifische Immunantwort gegen Infektionen bei Tier oder Mensch verstärkt.

## Beschreibung

Die Erfindung betrifft die Verwendung eines Extrakts aus einem phototrophen Mikroorganismus, insbesondere der Klassen Cyanophyceae, Rhodophyceae, Trebouxiophyceae, Chlorophyceae und Eustigmatophyceae in der Medizin, insbesondere als Adjuvans für Impfstoffe.

Bei der Haltung von Tieren verursachen pathogene Erreger häufig Erkrankungen, die zu Tierverlusten, Minderung der Wachstums- und Reproduktionsraten und zu wirtschaftlichen Einbußen führen. Zur Therapie werden Antibiotika eingesetzt, deren Verwendung wegen der wachsenden Gefahr der Resistenzausbildung und ihrer Nebenwirkungen auf Umwelt und Mensch zunehmend stärker reglementiert wird.

Eine Alternative ist die Gesunderhaltung der Tiere mit Hilfe prophylaktischer Schutzimpfungen gegen relevante Krankheitserreger.

Die Impfstoffe (Vakzine) werden aus den abgetöteten oder abgeschwächten Krankheitserregern oder deren Bestandteilen hergestellt. Ihre Verabreichung verursacht eine komplexe erregerspezifische Immunantwort, die von vielen Faktoren beeinflusst wird.

Im Stand der Technik ist bekannt, dass die Wirkung von Impfstoffen durch Adjuvantien verstärkt werden kann. Toxische Nebenwirkungen und Entzündungen limitieren jedoch ihren Einsatz. Bekannte vor allem in der Veterinärmedizin eingesetzte Adjuvantien sind Aluminiumhydroxid und Mineralöl, die jedoch häufig unerwünschte Nebenwirkungen verursachen. Daher besteht ein Bedarf an alternativen Adjuvantien ohne Nebenwirkungen bei gleichzeitiger Verstärkung insbesondere der humoralen Immunantwort.

### Beschreibung

Die vorliegende Erfindung betrifft Extrakte aus phototrophen Mikroorganismen, die vor allem die Effizienz der humoralen Immunantwort eines Wirts bei Zusatz zu Impfstoffen erhöhen. Die Extrakte werden als Adjuvans den Antigenen bzw. Vakzinen zugesetzt. Die Applikation der Vakzine erfolgt parenteral.

Unter dem Begriff "Immunstimulator" (Antigen) zur Generierung einer aktiven Immunität ist hier ein Stoff zu verstehen, der eine spezifische Immunantwort auslöst und die Bildung von Antikörpern, die gegen den verabreichten Immunstimulator gerichtet sind, induziert. Immunstimulatoren im Sinne der Erfindung sind Antigene, d. h. lebende oder tote pathogene Mikroorganismen, wie Bakterien, Viren oder Pilze oder deren Bestandteile.

Unter einem Adjuvans ist hierbei eine Substanz zu verstehen, die als Mischung mit einem Antigen (Vakzin) die Bildung von spezifischen insbesondere humoralen Antikörpern als Immunantwort auf dieses Antigen verstärkt.

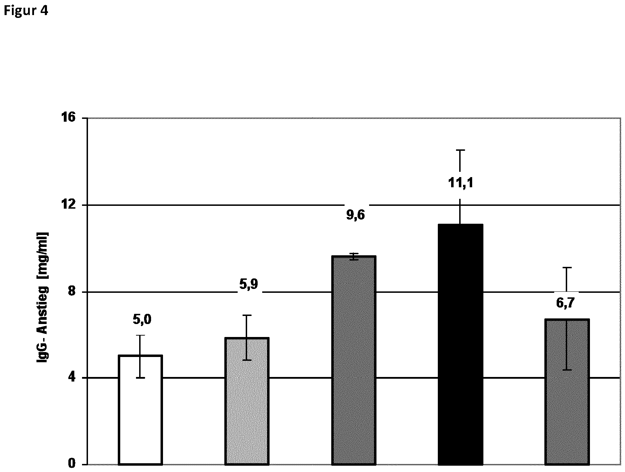

In einem ersten Aspekt betrifft die Erfindung die Verwendung eines Extrakts aus einem phototrophen Mikroorganismus in der Medizin, als Adjuvans für die Impfung gegen bakterielle, mukale und virale Infektionen und Erkrankungen. Die Immunstimulierung erfolgt bereits bei ein- bis zweimaliger Applikation, bevorzugt bei einmaliger Applikation (siehe Figur 4).

Der hier verwendete Begriff "phototropher Mikroorganismus" bezieht sich auf Organismen der Gruppen Chlorophyta, Rhodophyta, Heterokontophyta und Cyanobakterien, insbesondere der Klassen Cyanophyceae, Rhodophyceae, Trebouxiophyceae, Chlorophyceae und Eustigmatophyceae. Die "phototrophen Mikroorganismen" unterscheiden sich von heterotrophen Mikroorganismen durch ihren photosynthetischen Metabolismus. Die erfindungsgemäß verwendeten phototrophen Mikroorganismen sind für Mensch und Tier apathogen.

Der hier beschriebene Extrakt, d. h. der Auszug oder die Lösung von Wirkstoffen aus den phototrophen Mikroorganismen, ist bevorzugt ein wässriger oder wässrig-ethanolischer Extrakt. Der hier beschriebene Extrakt enthält keine lebensfähigen und keine vollständigen Zellen, sondern definierte organische und anorganische Zellbestandteile der ausgewählten Mikroorganismen. Er besteht allgemein aus komplexen wasserlöslichen Zellbestandteilen, wie Mono-, Di- und Polysacchariden, Proteinen und Aminosäuren sowie anorganischen Bestandteilen. Die Wirkstoffkomponenten liegen in einem Größenbereich kleiner 0,5 μm.

In einem zweiten Aspekt betrifft die Erfindung eine (pharmazeutische) Zusammensetzung, also eine Präparation, die eine Vakzine dargestellt, die als Komponenten mindestens einen Extrakt aus einem phototrophen Mikroorganismus und einen Immunstimulator (ein Antigen) zur Generierung einer aktiven, spezifischen, humoralen Immunantwort aufweist.

In einer Ausführungsform der Erfindung kann die Zusammensetzung neben dem hier beschriebenen Extrakt aus einem phototrophen Mikroorganismus auch mindestens ein weiteres Adjuvans aufweisen. Das weitere Adjuvans kann insbesondere Öl, Aluminiumhydroxid, komplettes Freund'sches Adjuvans, inkomplettes Freund'sches Adjuvans, stabilisierende kationische Peptide ausgewählt aus Polypeptiden, Protamin, Nukleolin, Spermin oder Spermidin, kationischen Polysacchariden, Chitosan, TDM, MDP, Muramyldipeptid, oder Alaun-Lösung, Pluronics und Lipopeptide, einschließlich Pam3Cys sein, die allesamt dem Fachmann bekannt sind. Wie eingangs erwähnt geht die Verwendung derartiger Adjuvantien jedoch häufig mit unerwünschten Nebenwirkungen einher, bspw. sehr schmerzhaften Reizungen und Entzündungen am Ort der Applikation des immunisierten Tieres oder Menschen.

Die Zusammensetzung kann optional einen pharmazeutisch geeigneten Träger und/oder weitere Hilfs- und Zusatzstoffe aufweisen.

Der hier verwendete Begriff "pharmazeutisch geeigneter Träger" umfasst bevorzugt einen oder mehrere kompatible flüssige Füllstoffe bzw. Verdünnungsmittel, welche für die parenterale Applikation geeignet sind. Die pharmazeutisch geeigneten Träger müssen selbstverständlich eine ausreichend hohe Reinheit und eine ausreichend geringe Toxizität aufweisen, um sie für die Applikation im human- oder veterinärmedizinischen Bereich geeignet zu machen. Einige Beispiele von Verbindungen, welche als pharmazeutisch geeignete Träger oder Bestandteile dienen können, sind Zucker, Öle, Emulgatoren, Antioxidantien; pyrogen-freies Wasser; isotonische Salzlösung und phosphatgepufferte Lösungen.

Die Wahl eines pharmazeutisch geeigneten Trägers wird grundsätzlich durch die Art bestimmt, durch die die erfindungsgemäßen pharmazeutischen Zusammensetzungen appliziert werden. Die erfindungsgemäßen pharmazeutischen Zusammensetzungen umfassen parenterale, einschließlich subkutane oder intravenöse Injektionen. Die geeignete Menge der zu verwendenden pharmazeutischen Zusammensetzung kann durch Routineexperimente mit Tiermodellen (z. B. Kaninchen, Schaf, Maus, Ratte, Hund und nichthumane Primatenmodelle) bestimmt werden. Bevorzugte Einheitsdosisformen zur Injektion schließen sterile Lösungen von Wasser, physiologischer Salzlösung oder Mischungen davon mit ein. Geeignete Träger zur Injektion schließen Hydrogele, Vorrichtungen zur kontrollierten oder verzögerten Freigabe, polylaktische Säure und Collagenmatrizen mit ein. Die pharmazeutisch geeigneten Träger zur Herstellung von Einheitsdosisformen sind im Stand der Technik bekannt.

Die Zusammensetzung gemäß der vorliegenden Erfindung umfasst typischerweise eine sichere und effektive Menge des erfindungsgemäßen Adjuvans. Eine "sichere und effektive Menge" ist eine solche Menge einer Substanz, die in Verbindung mit dem Antigen eine effektive spezifische humorale Immunantwort zur Bekämpfung von Infektionen und erregerbedingten Erkrankungen stimuliert. Gleichzeitig ist eine "sichere und effektive Menge" jedoch gering genug, um schwerwiegende Nebeneffekte zu vermeiden, also ein vernünftiges Verhältnis von Vorteil und Risiko zu ermöglichen. In Bezug auf das erfindungsgemäße Adjuvans bedeutet der Begriff "sichere und effektive Menge" bevorzugt eine solche Menge, die geeignet ist, das humorale Immunsystem in einer solchen Weise zu stimulieren, dass keine überschießenden bzw. schädlichen Immunreaktionen erzielt werden, jedoch bevorzugt auch keine solchen Immunreaktionen unterhalb eines messbaren Niveaus. Eine "sichere und effektive Menge" eines erfindungsgemäßen Adjuvans wird im Zusammenhang mit dem besonderen zu behandelnden Zustand variieren, sowie dem Alter und dem physischen Zustand des Patienten, der Dauer der Behandlung, der Natur der begleitenden Maßnahmen, des verwendeten besonderen pharmazeutisch geeigneten Trägers und ähnlichen Faktoren innerhalb des Wissens und der Erfahrung des begleitenden Arztes oder Tierarztes entsprechen. Die erfindungsgemäße Zusammensetzung kann für humane und wie auch für veterinärmedizinische Zwecke eingesetzt werden.

In einem dritten Aspekt betrifft die Erfindung die Verwendung einer Zusammensetzung der hier beschriebenen Art in der Medizin, insbesondere im Rahmen der Impfung als Adjuvans.

In einem vierten Aspekt betrifft die Erfindung ein Verfahren zur Herstellung einer Zusammensetzung nach Anspruch 1 bis 4, das die folgenden Schritte aufweist:
- Bereitstellen eines phototrophen Mikroorganismus,
- Herstellen eines wässrigen oder wässrig- ethanolischen Extraktes aus dem phototrophen Mikroorganismus,
- bevorzugt Abtrennung von Zellwandbestandteilen und Zellpartikeln,
- optional Fraktionierung und Konzentrierung des Extraktes,
- Mischen des Extrakts aus dem phototrophen Mikroorganismus mit einem oder mehreren Antigenen zur Generierung einer aktiven Immunantwort.

Das Antigen zur Generierung einer aktiven Immunität kann ein Bakterium, ein Virus oder ein Pilz bzw. deren Komponenten sein.

In einem fünften Aspekt betrifft die Erfindung ein Verfahren zur Immunisierung eines Tieres gegen eine erregerbedingte Infektion bzw. die von ihr hervorgerufenen Erkrankung. Erfindungsgemäß weist das Verfahren den Schritt der Applikation einer hierin beschriebenen Zusammensetzung auf.

Die Applikation kann gemäß der im Stand der Technik bekannten parenteralen Applikationsart erfolgen, z. B. durch, subkutane, intramuskuläre oder intravenöse Injektionen. Bevorzugt ist die subkutane Applikation.

Das bei dem Immunisierungsverfahren verwendete Tier ist in einer Ausführungsform der Erfindung ein Wirbeltier, bevorzugt ein Säugetier, ein Vogel oder Fisch. Als Säugetiere sind Nutz- bzw. Haustiere oder Menschen bevorzugt. Das Haustier kann beispielsweise ein Rind, Schwein, Pferd, Esel, Kamel, Katze, Hund Kaninchen, Ren, Huhn, Pute, Gans, Ente, das Nutztier ein Pelztier, z. B. Nerz, Nutria oder ein Fisch sein. Das zu impfende Tier kann auch ein Wildtier oder ein gezüchtetes bzw. in Gefangenschaft lebendes Tier (Zootier) wie beispielsweise ein Strauß, Lama oder Damwild sein.

Mit dem Immunisierungsverfahren kann eine Immunität sowohl gegen Infektionen insbesondere gegen bakterielle und virale, optional auch gegen mukale Infektionskrankheiten erreicht werden. Die bakterielle Infektionskrankheit kann sowohl durch gram-negative als auch durch gram-positive Erreger verursacht sein.

Mit dem hier beschriebenen Extrakt als Adjuvans kann durch Zumischung zu einem Antigen die Schutzwirkung der Impfstoffe praktisch gegen bakterielle als auch virale Infektionskrankheit der Wirbeltiere verbessert werden, beispielsweise Impfungen gegen die folgenden Infektionskrankheiten bzw. Erreger:
Influenza, SARS, Gelbfieber, AIDS, Condyloma acuminata, Dellwarze, Dengue-Fieber, Dreitagefieber, Ebolavirus, Frühsommermeningoenzephalitis (FSME), Gürtelrose, Hepatitis, Herpes-Simplex Typ I, Herpes-Simplex Typ II, Herpes zoster, Japanische Enzephalitis, Lassafieber, Marburg-Virus, Masern, Maul- und Klausenseuche, Mononukleose, Mumps, Norwalk-Virus-infektion, Pfeifersches-Drüsenfieber, Pocken, Polio (Kinderlähmung), Pseudokrupp, Ringelröteln, Tollwut, Warzen, West-Nil-Fieber, Windpocken, Zytomegalie-Virus (CMV), bakteriellen Infektionskrankheiten wie Lyme-Borreliose, bakterielle Meningitis, Anthrax, Appendizitis (Blinddarmentzündung), Botulismus, Campylobacter, Chlamydia trachomatis (Harnröhren-, Bindehautentzündung), Cholera, Diphtherie, Donavanosis, Epiglottitis, Fleckfieber, Flecktyphys, Gasbrand, Gonorhoe, Tularämie, Heliobacter pylori, Bordetella pertussis, klimatischer Bubo, Knochenmarksentzündung, Legionärskrankheit, Lepra, Listeriose, Mittelohrentzündung, Mycoplasma hominis, Neugeborenensepsis, Noma, Paratyphus, Pest, Reitersyndrom, Rocky Mountain spotted fever, Salmonellen-Paratyphus, Salmonellen-Typhus, Scharlach, Syphilis, Tetanus, Tripper, Tuberkulose, Typhus, Vaginitis (Kolpitis), Weicher Schanker; sowie E. coli-Infektionen und Intoxikationen (E. coli-Ruhr, E.coli-Enterotoxämie, E. coli-Septikämie), Salmonelleninfektionen (insbesondere S. cholera suis, S. dublin, S. gallinarum, S. pullorum, S. enteritis, S. thyphimurium), Streptokokkeninfektionen (Druse, Streptokokken-Infektionen beim Schwein, Sudden death-Syndrom bei Enten, Streptococcus zooepidemicuns-Infektion), Staphylokokkeninfektionen (Staph. hyicus-, Staph. aureus-, Staph. intermedius-Infektionen), Pasteurellen-Infektionen (Pasteurella multocida-Infektionen, Geflügelcholera, haemorrhagische Septicaemia, Wild-und Rinderseuche), Rhinitis atrophicans, Mannheimia-haemolytica-Infektion, Moraxella-Infektionen (infektiöse Keratoconjunctivitis infolge von Moraxella bovis-Infektion u. a.), Erysipelothrix rhusiopathiae-Infektion (Rotlauf, Septikämie beim Geflügel und bei Nagetieren), Moderhinke der Schafe (Bacteroides nodosus-Infektion, Fusobacterium necrophoruminfektion), Nekrobazillose der Tiere und des Menschen (Fusobacterium necrophorumilnfektion), Clostridien-Infektionen und -intoxikationen (Rauschbrand, Pararauschbrand, Botulismus, Tetanus, Cl. perfringens-Enterotoxämie, Gasbrand, ulzerative Enteritis der Vögel, Nekrotische Hepatitis, Bradsot, Cl. Spiriformes- Infektionen, Malignes Ödem, Nekrotisierende Mastitis, Bazilläre Hämoglobinurie), Bordetellose (Bordetella bronchiseptica-Infektion, Bordetella avium-Infektion), Riemerellose (R. anatipestifer-Infektion, R. columbina-Infektion), Hämophilose (Glässersche Krankheit, Haemophilus gallinarum-Infektion), Actinobacillus-pleuropneumoniae-Infektion der Schweine, Actinobacillus equuli-Infektion der Pferde, Chlamydien-Infektionen (Enzootischer Abort der Schafe, Chlamydieninfektionen der Rinder), Ornithobacterium rhinotracheale- Infektion, Mycoplasmen-Infektionen, Arcanobacterium pyogenes-Infektion, bakterielle Infektionen bei Fischen (Furunkulose/Aeromonas salmonicida-Infektion, Rotmaulkrankheit / Yersinia ruckeri-Infektion, Vibriosen (Vibrio anguillarum-Infektion).

Die erfindungsgemäße Zusammensetzung (Vakzine) kann einen Anteil des Extrakts aus einem phototrophen Mikroorganismus von 1 % bis 60 % (Gew.%) als Adjuvans enthalten, bevorzugt 5 % bis 30%, besonders 10% bis 20%. Weiterhin kann die Vakzine ein herkömmliches Adjuvans enthalten, wie Öl oder Aluminiumhydroxid. Das herkömmliche Adjuvans kann insbesondere einen Anteil von 1% bis 50% (Gew.%) an der Vakzine ausmachen.

In einem sechsten Aspekt betrifft die Erfindung ein Adjuvans, das durch Bereitstellen eines phototrophen Mikroorganismus und Herstellen eines wässrigen Extraktes aus dem phototrophen Mikroorganismus herstellbar ist. Bevorzugte Ausgestaltungen dieses Erfindungsaspekts ergeben sich aus der hier angegebenen Offenbarung.

In einem siebenten Aspekt betrifft die Erfindung einen Kit, der einen wässrigen Extrakt aus einem phototrophen Mikroorganismus und einen Immunstimulator (ein Antigen) zur Generierung einer aktiven, spezifischen, humoralen Immunantwort aufweist.

In einem achten Aspekt betrifft die Erfindung die Verwendung eines derartigen Kits in der Medizin im Rahmen einer parenteralen Impfung.

Der erfindungsgemäße Extrakt weist aufgrund seines biologischen Ursprungs, seiner Herstellungstechnologie und seiner Zusammensetzung bei erfindungsgemäßer Dosierung keine störenden Nebenwirkungen auf. Er bewirkt in Verbindung mit dem Antigen (Vakzin) eine Steigerung insbesondere der spezifischen humoralen Immunantwort. Wie sich aus den Beispielen ergibt, sind die Algenextrakte als Adjuvantien sehr wirksam und erhöhen die spezifische Resistenz gegen Infektionskrankheiten, was zur Verringerung der Tierverluste, Reduzierung der Leistungsminderung, Erhöhung der Zuwachsraten und verbesserten Zuchtergebnissen führt.

Die erfindungsgemäßen Extrakte aus einem phototrophen Mikroorganismus weisen typischer Weise einen Gehalt an Trockenmasse zwischen 0,1 bis 6,0 g pro 100 g (bevorzugt zwischen 0,2 bis 6,0 g pro 100 g) auf, mit einem pH-Wert von 4,0 bis 9,0 (bevorzugt einen pH-Wert von 4,0 bis 8,0) und einer Leitfähigkeit von 0,3 - 13,0 mS (Beispielspezifikation für Extrakt-Adjuvans aus Cyanophyceae siehe Tabelle 1).

In einem neunten Aspekt betrifft die Erfindung die Verwendung einer erfindungsgemäßen Zusammensetzung oder eines erfindungsgemäßen Kits zur Verstärkung der spezifischen humoralen Immunantwort auf ein appliziertes Antigen nach einmaliger parenteraler Applikation. Diese Verbesserung ist messbar in einem Anstieg des humoralen immunologischen Parameters Immunoglobulin G (IgG) (siehe Figur 4).

Die Erfindung wird nachfolgend anhand von Figuren und Beispielen demonstriert.

### Figuren

Es zeigen:

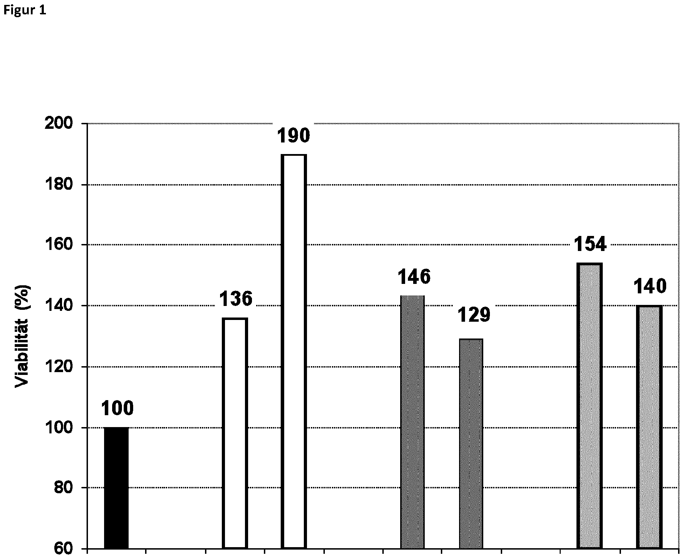

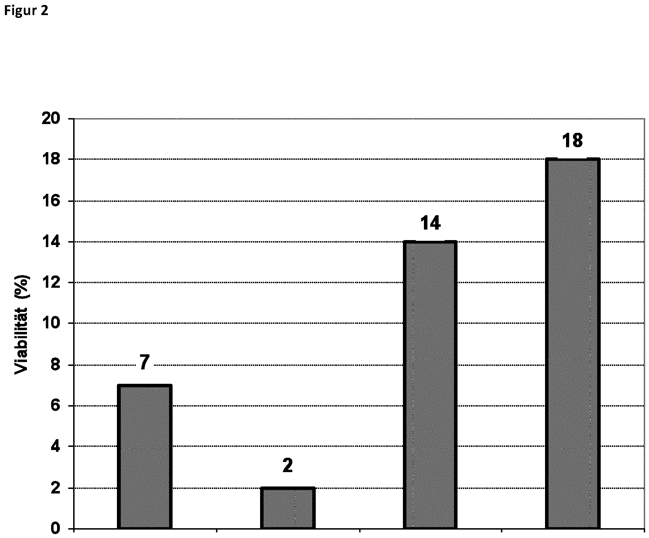

**Figuren 1** **und** **2****:** *In vitro* Prüfung von Algenextrakten (Algenadjuvantien) (Figur 1) und *in vitro* Prüfung von Impfstoffen (Antigene, bestehend aus folgenden pathogenen Bakterien: *Streptococcus sius, Pasteurella multiccida, Haemophilus parasuis und Bordetella bronchiseptica)* sowie Impfstoffen mit Adjuvantien (Figur 2) auf die Viabilität von Zellen.
   Die Charakterisierung der Adjuvantien (Extrakte) erfolgt an Schweineblut- Zellkulturen mittels eines in vitro MTT-Test. Unter definierten Bedingungen wird die Wirkung der Extrakte und der die Extrakte aufweisenden Vakzine auf die Zellaktivität und -vitalität lebender Blutzellen ermittelt.
   Gelbes Methyltetrazoliumsalz (MTT) wird durch zelluläre Dehydrogenasen zu blauem Formazan reduziert. Die Menge des gebildeten Farbstoffes korreliert mit der Zellaktivität und wird photometrisch ermittelt.
   Figur 1: *In vitro* Zellviabilität von Ferkel-Leukozyten in Abhängigkeit von Algenart, Dosierung und Präparation der Extrakte (MTT-Test)
   Figur 1 zeigt am Beispiel von Cyanophyceae-, Rhodophyceae-Extrakten und Extraktmischungen mit einem Trockenmassegehalt von 1,5 %, dass die erfindungsgemäßen Adjuvantien die Zellviabilität um bis zu 90 % gegenüber der Kontrolle (=100 %), d. h. den Blutzellen ohne Dosierung von Adjuvantien, steigern. Die Höhe der Steigerung der Zellaktivität ist abhängig von der Alge, der eingesetzten Extrakt-Konzentration (Verdünnung z. B. ein Teil Algenextraxt in 22 Teilen Zellkulturmedium) und der Präparation der Adjuvantien (mit und ohne Diafiltration). (Balken von links nach rechts: 1 Kontrolle = unbehandelte Leukozyten; 2 Cyanophyceae- Extrakt, Verdünnung 1:22; 3 Cyanophyceae-Extrakt, Verdünnung 1:52; 4 Rhodophyceae- Extrakt, Verdünnung 1:24; 5 Rhodophyceae-Extrakt, diafiltriert, Verdünnung 1:42; 6 Extrakt-Mix1 (Mischung wässriger Extrakte der Mikroalgenklassen Rhodophyceae und Eustigmatophyceae), Verdünnung 1:52; 7 Extrakt-Mix2 (Mischung wässriger Cyanophyceae - und *Ascophyllum-* Extrakte), Verdünnung 1:12.
Figur 2: *In vitro* Zellviabilität von Ferkel- Leukozyten nach Dosierung von Impfstoffen ohne bzw. mit Öl-Adjuvans bzw. Algenextrakt-Adjuvantien (MTT-Test)
   Figur 2 zeigt ein Beispiel für einen MTT-Test mit Vakzinen, mit und ohne erfindungsgemäße Adjuvantien. Das Beispiel für das Adjuvans "Cyanophyceae-Extrakt" veranschaulicht, dass die Vakzine ohne Adjuvans und mit handelsüblichem Öl-Adjuvans eine deutlich schlechtere Wirkung auf die Viabilität der Ferkel-Leukozyten hatten. Die beste Wirkung auf die Zellvialbilität wurde in diesem Beispiel mit der Dosierung von 20 % Algenextrakt-Adjuvans im Vakzin erreicht. (Balken von links nach rechts: 1 Kontrolle ohne Adjuvans; 2 Impfgruppe Antigen mit 20 % Öl-Adjuvans; 3 Impfgruppe Antigen mit 10 % Cyanophyceae-Extrakt; 4 Impfgruppe Antigen mit 20 % Cyanophyceae- Extrakt).

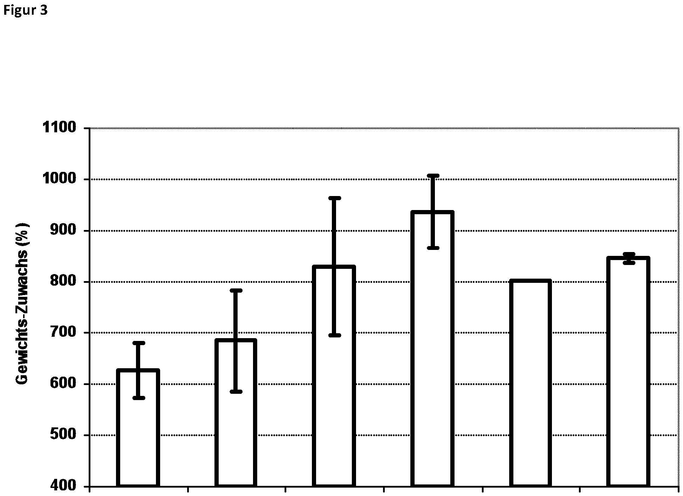

**Figur 3** zeigt die Effekte der einmaligen Impfungen mit Antigenen und Adjuvantien auf den prozentualen Zuwachs von Ferkeln von dem Tag der Impfung bis zur Schlachtung (Einstallgewicht = 100 %).
   Balken von links nach rechts: 1 Kontrolle, ungeimpft; 2 geimpft: Antigen ohne Adjuvans; 3 geimpft: Antigen und 20% kommerzielles Öl-Adjuvans; 4 geimpft: Antigen und Kombination von 10 % Cyanophyceae-Extrakt-Adjuvans und 10 % Öl- Adjuvans; 5 geimpft: Antigen und 20% Cyanophyceae-Extrakt-Adjuvans; 6 geimpft: Antigen und 10% Cyanophyceae-Extrakt-Adjuvans.
   Im Vergleich zur Kontrollgruppe (ungeimpft) erreichten alle geimpften Versuchsgruppen höhere Zuwachsraten. Die beste Variante in diesem Beispiel war: Antigen und Kombination von 10 % Cyanophyceae-Extrakt-Adjuvans und 10 % Öl-Adjuvans; also eine Mischung von Antigen, erfindungsgemäßen Algenextrakt-Adjuvans und Öl-Adjuvans.
**Figur 4****:** Einfluss erfindungsgemäßer Adjuvantien aus phototrophen Mikroorganismen nach einmaliger Vakzinierung auf die humorale Imunantwort am Beispiel des IgG-Anstiegs (mg IgG/ml Serum) bei Ferkeln im Zeitraum vom Tag der Impfung bis zum 28. Tag nach der Impfung
   Balken von links nach rechts: 1 Kontrolle, ungeimpft; 2 geimpft: Antigen mit 10 % Öl-Adjuvans; 3 geimpft: Antigen mit 10 % Cyanophyceae-Extrakt; 4 geimpft: Antigen mit 10 % Rhodophyceae-Extrakt; 5 geimpft: Antigen mit 10 % Trebouxiophyceae-Extrakt.
Figur 4 zeigt ein Beispiel für die Wirkung der Impfungen auf den humoralen immunologischen Parameter Immunglobulin G (IgG). Der Anstieg des IgG wurde mittels ELISA-Tests (Enzym-Linked-Imunnosorbent-Assay) ermittelt und quantifiziert. Bei allen geimpften Gruppen war bis zum 28. Tag nach der Impfung ein stärkerer Anstieg des IgGs als in der Kontrollgruppe zu beobachten. Der stärkste Anstieg wurde in der Versuchs-Gruppe "Adjuvans-Extrakt aus Mikroorganismen der Klasse Rhodophyceae " gemessen.

### Beispiele

Durch die parenterale Applikation von Extrakten aus phototrophen Mikroorganismen in Kombination mit bestandsspezifischen Immunstimulatoren (Antigenen) für Schweine, d. h. mit Vakzinen basierend auf einem Mikroorganismenspektrum, das dem potentiellen spezifischen Krankheitsgeschehen im Tierbestand entspricht, wurde die spezifische humorale Immunantwort gesteigert, die Zuwachsrate der Tiere verbessert und das Krankheitsgeschehen im Bestand reduziert.

Die Effekte der Impfungen wurden durch *in vitro* und *in vivo* Untersuchungen, z. B. mittels Zelltests, immunologische Untersuchungen und an Hand von Tierzuwachsraten nachgewiesen.

### Verwendete phototrophe Mikroorganismen

Für die Extraktherstellung wurden phototrophe Mikroorganismen der Klassen Cyanophyceae, Rhodophyceae, Trebouxiophyceae, Chlorophyceae und Eustigmatophyceae der Gattungen *Spirulina, Chlorella, Porphyridium, Nannochloropsis, Galderia, Cyanidium, Nostoc, Scenedesmus, Kirchneriella, Dunaliella* und *Phormidium* eingesetzt.

### Kultivierung

Die Mikroalgen wurden unter Einhaltung der nachstehend aufgeführten Kultivierungsstufen unter speziellen kontrollierten Bedingungen im "scale up" kultiviert:
- Aktivierung des Stammes durch Überimpfen in stamm- (art-) spezifischem Flüssigmedium.
- Scale up von 10 ml über 50, 100, 500 bis zur 2.000 ml Laborkultivierung unter Steuerung von Temperatur, Bestrahlung, CO₂₋ Luft- Begasung und mechanische Durchmischung.
- Scale up zum kleintechnischen Maßstab 30, 100 bis zum 4.000 Liter-Maßstab mit on-und off-line Messung und Regelung von Temperatur, pH-Wert, optische Dichte, CO₂-Dosierung und Pumpen-Frequenz.

### Kultivierungsbedingungen:

*1. Chlorella spp.:* ½ Tamiya Medium; 20 - 35 °C; pH 7,0 - 7,5; in Abhängigkeit von der Wachstumsphase 50 ->1500 μE/(m²*s); 3% CO₂- Luftgemisch- Begasung, mit De-Immobilisierungsgranulaten bei maximaler Pumpendrehzahl.
*2. Spirulina spp.:* Zarrouk Medium; 25°C - 30°C; pH 8,0 - 10; ca. 20 - 1500 μE/(m²*s); Luft-Begasung, mit De-Immobilisierungsgranulaten bei reduzierter Pumpendrehzahl.

Die speziellen Kultivierungsbedingungen zeichnen sich im Weiteren dadurch aus, dass durch die Kultivierung eine biologische Anreicherung der Biomasse mit bestimmten Mineralstoffen wie Zn, Se, Ca u. a. erfolgt. Das wird durch Modifizierung der Nährlösung erreicht. Die Modifizierung kann die zusätzliche Dosierung von Salzen dieser Mineralien beinhalten.

Die Ernte der Algenbiomasse erfolgt durch Filtration, Separation oder Zentrifugation einschließlich Waschvorgänge. Die Waschung kann mit Wasser, physiologischer Kochsalzlösung, sauren oder alkalischen wäßrigen Lösungen erfolgen. Es kann sich eine Frostung oder Trocknung der Biomasse mittels schonender Sprüh-, Gefrier- oder Sonnentrocknung und eine Vakuumverpackung anschließen.

Im allgemeinen ist die Kultivierung von photoprophen Mikroorganismen dem Fachmann bekannt und ist u. a. in der folgenden Literatur beschrieben:
Richmond, A., et. al, 2004, Handbook of: Microalgal Culture Biotechnology and Applied Phycology, Blackwell Publishing, S.566 ff.
Iwamoto, H., 2004, Industrial Production of Microalgal Cell-mass and Secondary Products - Major Industrial Species *Chlorella,* In: Richmond, A., et. al, 2004. Handbook of: Microalgal Culture Biotechnology and Applied Phycology. Blackwell Publishing, 255-263.
Becker, E.W., 1994, Microalgae: Biotechnology and Microbiology, Cambridge UniversityPress, S. 301 ff.
Cohen, Z., 1999, Chemicals from Microalgae, Crc Pr Inc, 1-24, 282-291, 41-56, 261-281, 292-312.
   http://www.fao.org/docrep/003/w3732e/w3732e06.htm (Website der "Food and Agriculture Organisation of the United Nations".)

### Gewinnung von Extrakten - Herstellung und Charakterisierung

Die Wirkstoffisolate werden durch Extraktionen von Biomassen gewonnen. Die Extraktion kann mit Wasser oder Ethanol-Wasser-Gemisch erfolgen. Sie beinhalten das definierte Rehydratisieren (bei definierter Konzentration, Temperatur, Dauer, Rührgeschwindigkeit), Erhitzen, Kochen, Separieren, Filtrieren, Aufkonzentrieren durch Ultra- und evt. Diafiltration, Sterilisieren und evt. Lyophilisieren. Derartige Verfahren sind dem Fachmann bekannt und werden u. a. in der folgenden Literatur beschrieben:
Molina Grima, E., et, al, Downstream Processing of Cell-mass and Products, In: Richmond, A., et. al, 2004. Handbook of: Microalgal Culture Biotechnology and Applied Phycology. Blackwell Publishing, 230-239.
Heiss, R., 2004, Lebensmitteltechnologie: Biotechnologische, chemische, mechanische und thermische Verfahren der Lebensmittelverarbeitung, Springer, Berlin, 6. Auflage, 136-138, 425-426.
Salzer, U.-J., Fred Siewek, F., 2010, Handbuch Aromen und Gewürze, Behr's Verlag Bd. 1, 3A, 5-20.
Ziegler; E., 1982, Die natürlichen und künstlichen Aromen, Dr. Alfred Huthig Verlag, Heidelberg, 221-242.
Kingsley S. Rowan, 1989, Photosynthetic Pigments of Algae, Cambridge University Press, 192-194.
Die Herstellungstechnologie ist artspezifisch und dem Fachmann entweder bekannt oder kann anhand seines allgemeinen Fachwissens mittels Routineversuchen ermittelt werden.

Die Gewinnung der Wirkstoffisolate stellt sich typischerweise folgendermaßen dar:
- 90-98 % Wasser bzw. 12-20%Ethanol und 70-78% Wasser (18-20 °C) vorlegen und 2-10 % Algenbiomasse einrühren
- 1-14 Stunden unter Rühren rehydratisieren,
- erhitzen auf 60 °C bis 98 °C, bevorzugt auf 90 bis 98 °C,
- 1-2 Stunden unter Rühren extrahieren,
- Abtrennen der groben Feststoffe durch Separation oder Zentrifugieren,
- weitere Reinigung durch Mikrofiltration mit 0,45 μm Filter bei 20 °C
- Aufkonzentrieren durch Ultrafiltration, Einstellen der Trockenmassegehalte
- Stabilisieren durch Sterilfiltrieren, Autoklavieren oder Konservieren

Es werden definierte Extrakte erhalten. Das Molekulargewicht der Algenwirkstoffe in den Extrakten ist größer als 1000 Dalton.

Die Extrakte werden physikalisch, chemisch, mikrobiologisch und biologisch analysiert und mit Spezifikationen und Datenblättern charakterisiert (Tabelle 1).

### Herstellung des Vakzins

Die Vakzine werden aus Antigenen d.h. lebenden oder toten pathogenen Bakterien, Viren und Pilzen oder deren Bestandteilen, Flüssig- oder Trocken-Algenextrakt als Adjuvans, gegebenenfalls physiologischer Kochsalzlösung, gegebenenfalls anderen Adjuvantien wie Öl oder Al(OH)₃ und weiteren Bestandteilen hergestellt.

Die Dosierung des Algenextrakt-Adjuvans im Vakzin beträgt bis 60 %, bevorzugt bis zu 30%, besonders bevorzugt bis zu 20 %.

**Tabelle 1: Spezifikation für Adjuvantien aus phototrophen Mikroorganismen der Klasse Cyanophyceae (Extrakte nach wässriger bzw. wässrig-ethanolischer Extraktion)**

| **Parameter** | **Methode/Gerät** | **Meßwerte/Bereich** |
|---|---|---|
| Trockenmasse | DAB 10 (V.6.22.N2) | |
| | Trockernmassebestimmer bei 105°C | 0,1 - 6,0 g/ 100g |
| pH-Wert | DAB 10 (V.6.3.1) | |
| | Potentiometer | 4,0 - 9,0 |
| Leitfähigkeit | Condictivitymeter FE30/ FG3 Mettler Toledo | 0,3 - 13 mS |
| Refraktionsindex | DAB 10 (V.6.5) | |
| | Refraktometer (20 °C) | 1,3 - 1,4 |
| LAB-Farbwert L*a*b* | Spektrale Farbmessung nach CIE | L*=40-60 |
| | (DIN 5033, DIN 6174) | a*=-1,0 bis -5,0 |
| | Farbmessgerät LUCI 100, Dr. Lange | b*= 15,0 - 25,0 |
| | | |
| Protein | DAB 10 (V.3.5.2) | |
| | Kjeldahl-Bestimmer | 0,6 - 2,0 g/100g |
| Mineralstoffe | DAB 10 (V.3.2.16) Veraschung, Muffelofen | 1,45 - 4,0 g/100g |
| Kohlenhydrate | HPLC | 0,15 - 1,0 g/100g |
| | | |
| Rhamnose | HPLC | 3,5 - 63,5 mg/100g |
| Galactose | HPLC | 30 - 105 mg/100g |
| Xylose | HPLC | 3,0 - 25 mg/100g |
| Glucose | HPLC | 1,5 - 5,5 mg/100g |
| | | |
| s | Röntgenfluoreszenzanalyse (RFA) | 18 - 41 mg/100g |
| Ca | Röntgenfluoreszenzanalyse (RFA) | 2,0 - 7,0 mg/100g |
| Zn | Röntgenfluoreszenzanalyse (RFA) | 2,1 - 160 μg/100g |
| | | |
| **Mikrobiologie** | | |
| Sterilität | Sterilitätskontrolle nach EU AB | steril |
| | Ph. Euro 6.0/2.06.12; 13.00 | |

## Patentansprüche

1. Zusammensetzung, aufweisend:
- einen Extrakt aus einem phototrophen Mikroorganismus und
- einen Immunstimulator zur Generierung einer aktiven Immunität.

2. Zusammensetzung nach Anspruch 1, wobei der phototrophe Mikroorganismus ausgewählt ist aus den Klassen Cyanophyceae, Rhodophyceae, Trebouxiophyceae, Chlorophyceae und Eustigmatophyceae.

3. Zusammensetzung nach Anspruch 2, wobei der phototrophe Mikroorganismus ausgewählt ist aus den Gattungen *Spirulina, Chlorella, Porphyridium, Nannochloropsis, Galderia, Cyanidium, Nostoc, Scenedesmus, Kirchneriella, Dunaliella* und *Phormidium.*

4. Zusammensetzung nach Anspruch 1 bis 3, weiterhin aufweisend eine Substanz, die ausgewählt ist aus der Gruppe bestehend aus: Öl, Aluminiumhydroxid, komplettem Freund'schen Adjuvans, inkomplettem Freund'schen Adjuvans, stabilisierenden kationischen Peptiden ausgewählt aus Polypeptiden, Protamin, Nukleolin, Spermin oder Spermidin, kationischen Polysacchariden, Chitosan, TDM, MDP, Muramyldipeptid, sowie Alaun-Lösung, Pluronics und Lipopeptiden, einschließlich Pam3Cys.

5. Verwendung einer Zusammensetzung nach Anspruch 1 bis 4 in der Medizin, insbesondere zur Impfung (parenteralen Applikation).

6. Verwendung nach Anspruch 5 zur Verstärkung der spezifischen humoralen Immunantwort nach einmaliger parenteraler Applikation.

7. Verfahren zur Herstellung einer Zusammensetzung nach Anspruch 1 bis 4, aufweisend die folgenden Schritte:
- Bereitstellen eines phototrophen Mikroorganismus,
- Herstellen eines Extraktes aus dem phototrophen Mikroorganismus
und
- Mischen des Extrakts aus dem phototrophen Mikroorganismus mit einem Immunstimulator zur Generierung einer aktiven Immunität.

8. Verwendung eines Extrakts aus einem phototrophen Mikroorganismus in der Medizin als Adjuvans.

9. Verwendung nach Anspruch 8, wobei der phototrophe Mikroorganismus ausgewählt ist aus den Klassen Cyanophyceae, Rhodophyceae, Trebouxiophyceae, Chlorophyceae und Eustigmatophyceae.

10. Verwendung nach Anspruch 9, wobei der phototrophe Mikroorganismus ausgewählt ist aus der Gruppe bestehend aus den Gattungen *Spirulina, Chlorella, Porphyridium, Nannochloropsis, Galderia, Cyanidium, Nostoc, Scenedesmus, Kirchneriella, Dunaliella* und *Phormidium.*

11. Kit, aufweisend
- einen Extrakt aus einem phototrophen Mikroorganismus und
- einen Immunstimulator zur Generierung einer aktiven Immunität.

12. Verwendung eines Kits nach Anspruch 11 in der Medizin als Adjuvans zur parenteralen Vakzinierung.

13. Verwendung nach Anspruch 12 zur Verstärkung der spezifischen humoralen Immunantwort nach einmaliger parenteraler Applikation.
